# EUROPÄISCHE PATENTSCHRIFT

(11) **EP 2 508 140 B1**
(45) Veröffentlichungstag und Bekanntmachung des Hinweises auf die Patenterteilung: **25.12.2013**
(21) Anmeldenummer: 11161512.6
(22) Anmeldetag: 07.04.2011
(51) Int. Cl.: A61B 17/28, A61B 19/00

(54) **Zerlegbare chirurgische Zange**
Dismountable surgical forceps
Pince chirurgicale démontable

(43) Veröffentlichungstag der Anmeldung: 10.10.2012
(73) Patentinhaber: Waldemar Link GmbH & Co. KG, 22339 Hamburg (DE)
(72) Erfinder: Bartels, Carolin, 25355 Barmstedt (DE); Dmuschewsky, Klaus, 22397 Hamburg (DE)
(74) Vertreter: Glawe, Delfs, Moll

(56) Entgegenhaltungen:
- DE-A1-102009 003 273
- US-A- 168 012
- US-A- 1 475 569
- US-A- 2 632 661
- US-A- 5 197 879

## Beschreibung

Die Erfindung betrifft eine zerlegbare chirurgische Zange, die ein Drehgelenkelement mit einem Verdickungselement aufweist.

Werkzeuge mit Greif- oder Schneidfunktion wie z. B. Scheren oder Zangen werden in vielen Bereichen eingesetzt. In der Regel bestehen diese Werkzeuge aus zwei Teilen, die über eine Schraube miteinander verbunden sind. Möchte man diese beiden Teile reinigen, was unter anderem in der medizinischen Anwendung nötig ist, muss die Schraube gelöst werden. Des Weiteren muss das chirurgische Besteck die Anforderungen der Operation erfüllen. Das Besteck, insbesondere chirurgische Zangen, muss stabil, funktional und leicht zu handhaben sein. Weiter sollte es eine federnde Endstellung aufweisen, um die Kraft auf die gehaltenen Objekte zu begrenzen. Ein Teil der leichten Handhabung ist das schnelle und einfache Auseinanderbauen des chirurgischen Bestecks. Dies ist notwendig, damit das chirurgische Besteck in einem Autoklaven desinfiziert und gereinigt werden kann.

Der übliche Aufbau von chirurgischen Scheren oder Zangen umfasst viele Kleinteile, wobei die Zangenteile durch Schraub- oder Nagelverbindungen zusammengehalten werden. Vor der Desinfektion und Reinigung muss das Besteck umständlich auseinandergebaut werden. Nach der Desinfektion und Reinigung muss das chirurgische Besteck wieder umständlich zusammengebaut werden. Wenn Kleinteile verloren gegangen sind, ist der Zusammenbau zunächst nicht mehr möglich, und das Besteck steht für die nächste Operation nicht mehr zur Verfügung. Aus diesem Grund wurden Werkzeuge entwickelt, die einfach in ihre Einzelteile zerlegt werden können. Diese Werkzeuge umfassen zwei Einzelteile, die ohne weiteres Werkzeug lösbar sind.

Die US-Patentschrift US 2,632,661 offenbart ein Drehgelenk für eine Schere oder eine Zange, wobei ein Gelenkteil eine Führungsschiene umfasst, die in eine Führungsnut des anderen Gelenkteils angekoppelt werden kann. Die Kopplung der beiden Gelenkteile ist nur bei einem bestimmten Öffnungswinkel des Gelenks möglich. Bei diesem Öffnungswinkel werden die Führungsschienen des einen Gelenkteils nicht mehr von der Führungsnut des anderen Gelenkteils umfasst, wobei die beiden Gelenkteile in dieser Position gelöst werden können. Der Nachteil dieser Vorrichtung ist, dass die Führungsnuten nur sehr schlecht gereinigt werden können, da sie sehr tief und schmal sind. So können sich z. B. Gewebeteile oder Gewebeflüssigkeiten in diesen Nuten festsetzen, so dass sich dort Bakterien und Viren vermehren können bzw. diese Bakterien und Viren auch in einem desinfizierenden Autoklaven nicht abgetötet werden können. Weiter weist diese Vorrichtung den Nachteil auf, dass beim weiten Öffnen die beiden Gelenkteile unbeabsichtigt entkoppelt werden. Dies erschwert die Handhabung des Werkzeugs und kann bei chirurgischen Eingriffen auch ein Sicherheitsrisiko darstellen.

Die US-Patentschrift US 5,197,879 formt die Basis für den Oberbegriff des Anspruchs 1 und offenbart ein Drehgelenk für scherenartige Werkzeuge im medizinischen Bereich, das aus zwei identisch ausgeformten Hälften besteht. Jede Hälfte umfasst eine Führungsausnehmung und eine Führungsschiene, wobei im gekoppelten Zustand die Führungsschiene des einen Teils in die Führungsnut des anderen Teils eingreift. Das Drehgelenk kann in einer bestimmten Öffnungsposition entkoppelt werden, wobei die Führungsschienen in dieser Öffnungsposition nicht mehr in die Führungsnuten eingreifen. Der Nachteil dieser Vorrichtung ist, dass es ebenfalls zu einer unbeabsichtigten Entkoppelung beim weiten Öffnen kommen kann.

Demnach besteht die Aufgabe darin, eine Zange oder Schere mit einem zerlegbaren Drehgelenk bereitzustellen, die keine Kleinteile aufweist, möglichst wenige Einzelteile aufweist, leicht zu handhaben ist und die oben beschriebenen Nachteile vermeidet.

Die Aufgabe wird erfindungsgemäß gelöst durch eine Zange oder Schere mit den Merkmalen des unabhängigen Anspruchs. Vorteilhafte Weiterbildungen sind Gegenstand der abhängigen Ansprüche.

Demnach betrifft die Erfindung eine Zange, die einen Griff, ein Drehgelenk und ein Maul aufweist. Weiter weist sie zwei Zangenteile auf, die jeweils zwei identisch ausgeführte, lösbar gekoppelte, zapfenlose Drehgelenkelemente aufweisen, wobei das erste Drehgelenkelement eine erste Führungsschiene und eine erste Führungsausnehmung aufweist, und das zweite Drehgelenkelement eine zweite Führungsschiene und zweite Führungsausnehmung aufweist, wobei die in einen Hinterschnitt der Führungsausnehmungen an den Drehgelenkelementen greifenden Führungsschienen eine Gleitführung für das Drehgelenk bilden. Das erste Drehgelenkelement weist ein erstes Verdickungselement auf und das zweite Drehgelenkelement weist ein zweites Oberflächenelement auf, wobei die Drehgelenkelemente im gekoppelten Zustand gegeneinander zwischen einer Endposition und einer Sicherungsposition um eine zentrale Drehachse drehbar gelagert sind. In der Sicherungsposition wirkt das erste Verdickungselement mit dem zweiten Oberflächenelement so zusammen, dass die beiden Drehgelenkelemente verklemmt sind. Dabei kann die Verklemmung durch einen erhöhten Kraftaufwand überdrückt werden.

Die Erfindung ermöglicht eine einfache Trennung der Zangenteile und benötigt dazu keine Kleinteile wie Schrauben oder Nägel. Sie ist in zwei Teilen realisierbar und weist ein Drehgelenk auf, das keinen Zapfen aufweist. Dadurch wird eine effektive und einfache Desinfektion und Reinigung z. B. in einem Autoklaven ermöglicht. Die Führungsausnehmung kann sehr flach und breit gestaltet werden, so dass im medizinischen Bereich Gewebereste und Gewebeflüssigkeitsreste leicht entfernt werden können. Dadurch wird Bakterien und Viren die Möglichkeit genommen, sich einer Desinfektion zu entziehen. Durch das Verklemmen des Verdickungselements des einen Drehgelenkelementes mit dem Oberflächenelement des anderen Drehgelenkelementes wird ein unbeabsichtigtes Öffnen und Entkoppeln der beiden Drehgelenkelemente verhindert. Dies erleichtert die Handhabung des Werkzeugs erheblich. Auf diese Weise verbindet die Erfindung den Vorteil der Entkoppelbarkeit der beiden Zangenteile mit einer sicheren Arbeitsweise.

Die Vorrichtung weist eine Öffnungsposition auf, in der das erste und das zweite Drehgelenkelement voneinander gelöst bzw. miteinander gekoppelt werden können. In dieser Öffnungsposition ist keine Führungsschiene mehr in einer Führungsausnehmung angeordnet. Durch eine Drehung um die zentrale Achse greifen die Führungsschienen in die Führungsausnehmung ein und koppeln das erste und das zweite Drehgelenkelement miteinander. Weiter wird die Vorrichtung dadurch in die Sicherungsposition gebracht. In der Sicherungsposition verklemmen das erste und das zweite Verdickungselement mit dem ersten bzw. zweiten Oberflächenelement. Diese Verklemmung lässt sich durch weitere Drehung mit erhöhtem Kraftaufwand überwinden. Durch weitere Drehung gelangt die Vorrichtung in die Endposition. In der Endposition ist das Maul der Zange bzw. sind die Klingen der Scheren geschlossen. Aus der Endposition kann die Vorrichtung nur so weit geöffnet werden, bis die Sicherungsposition erreicht ist.

Die Drehgelenkelemente sind vorzugsweise federnd ausgeführt. Bei geschlossenem Maul sowie während des Haltens eines Gegenstands im Maul nehmen die Drehgelenkelemente einen Teil der auf die Bedienelemente ausgeübten Kraft auf.

Die beiden Drehgelenkelemente weisen vorzugsweise jeweils eine geschlitzte Ringfeder auf. Die Achse der Ringfeder fällt dabei mit der Drehachse des Drehgelenks zusammen. Der Schlitz der Ringfeder ist auf der Seite angeordnet, an der die Bedienelemente des Werkzeugs angeordnet sind. Die Ringfedern entfalten ihre Wirkung in der Endposition. Werden die Bedienelemente in dieser Position betätigt, in der das Maul der Zange geschlossen ist, wird der Schlitz der Ringfeder zusammengepresst und die Kraft wird auf die Ringfeder übertragen. Dadurch wird eine überhöhte Kraftübertragung von den Bedienelementen auf das geschlossene Maul einer Zange verhindert.

Hält das Maul einen Gegenstand, so wird ein Teil der auf die Bedienelemente ausgeübten Kraft ebenfalls auf die Ringfeder übertragen. Die erlaubt dem Benutzer durch eine leichte Erhöhung des Kraftaufwandes auf das Bedienelement zu prüfen, ob der zu ergreifende Gegenstand von dem Maul bereits umspannt ist.

Durch diese taktile Rückmeldung kann eine Beschädigung der durch eine Zange gehaltenen Objekte vermieden werden. Dies ist besonders im medizinischen Bereich von Vorteil, wenn mit einer Zange z. B. Nadeln zum Vernähen von Wunden gehalten werden sollen. Weiter hat die Ausführungsform mit der Ringfeder den Vorteil, dass bei einer Drehung weniger Reibung zwischen den beiden Drehgelenkelementen auftritt als bei einer Ausführungsform ohne Ringfeder. Dies erhöht die Leichtgängigkeit des Werkzeugs erheblich. Weiter werden zusätzliche Oberflächen vermieden, die gereinigt werden müssen.

Durch eine identische Ausführung beider Zangenteile wird eine kostengünstige Herstellung ermöglicht, da die Anzahl der verschiedenen Teile gering gehalten wird. Weiter kann bei einer Beschädigung oder bei einem Verlust des einen Zangenteils das andere Zangenteil weiterhin genutzt werden, was für den Anwender den Vorteil hat, dass die Zange auch bei eventuellem Verlust oder Beschädigung eines Zangenteils kostengünstig weiter genutzt werden kann.

Vorzugsweise werden die Bedienelemente mit Ausnehmungen ausgestattet, in denen während der Benutzung die Finger positioniert werden können. Dadurch wird ein Abrutschen der Finger verhindert. Auf diese Weise erhöht sich die Handhabbarkeit des Werkzeugs.

In einer ersten Ausführungsform der Erfindung ist die Führungsschiene auf dem Rand der Ringfeder angeordnet. Das Verdickungselement ist ebenfalls auf dem Rand der geschlitzten Ringfeder angeordnet. Weiter ist das Verdickungselement getrennt von der Führungsschiene angeordnet. Die Führungsausnehmung ist in dieser Ausführungsform in zwei Teilen am Maulelement und am Bedienelement angeordnet.

In einer weiteren Ausführungsform der Erfindung sind die Führungsausnehmung und das Verdickungselement auf dem Rand der Ringfeder angeordnet. In dieser Ausführungsform ist die Führungsschiene in zwei Teilen am Maulelement und am Bedienelement angeordnet.

In einer weiteren Ausführungsform der Erfindung können die Führungsschiene oder die Führungsausnehmung ein Verdickungselement aufweisen. Das Verdickungselement ist dabei so angeordnet, dass es in der Sicherungsposition mit dem Oberflächenelement des anderen Drehgelenkelements zusammenwirkt und eine Verklemmung bildet.

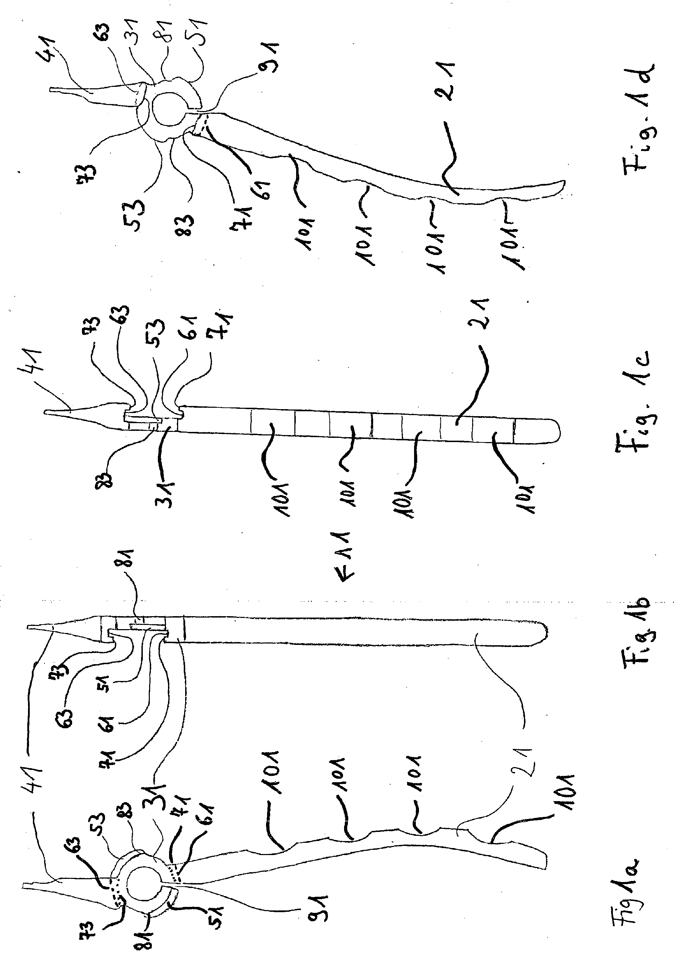

Die Erfindung wird im Folgenden beispielhaft unter Bezugnahme der beigefügten Zeichnungen anhand einer vorteilhaften Ausführungsform detailliert erläutert. Hierbei zeigen
- Fig. 1 a-d: ein erstes Zangenteil in Ansichten von vorne, von links, von rechts und von hinten,

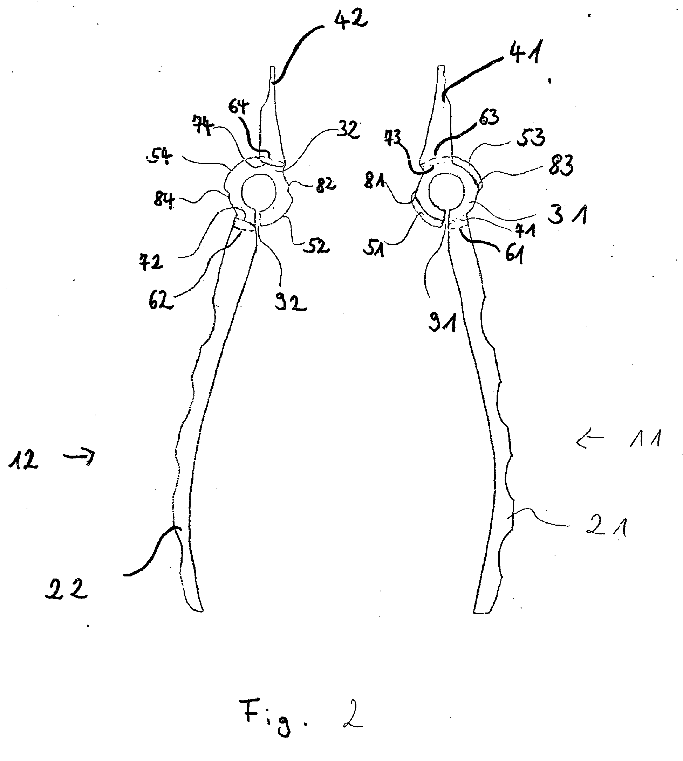

- Fig. 2: voneinander gelöste Zangenteile,
- Fig. 3: die beiden Zangenteile in der Öffnungsposition,
- Fig. 4: die beiden Zangenteile in der Sicherungsposition,
- Fig. 5: die beiden Zangenteile in der Endposition, und

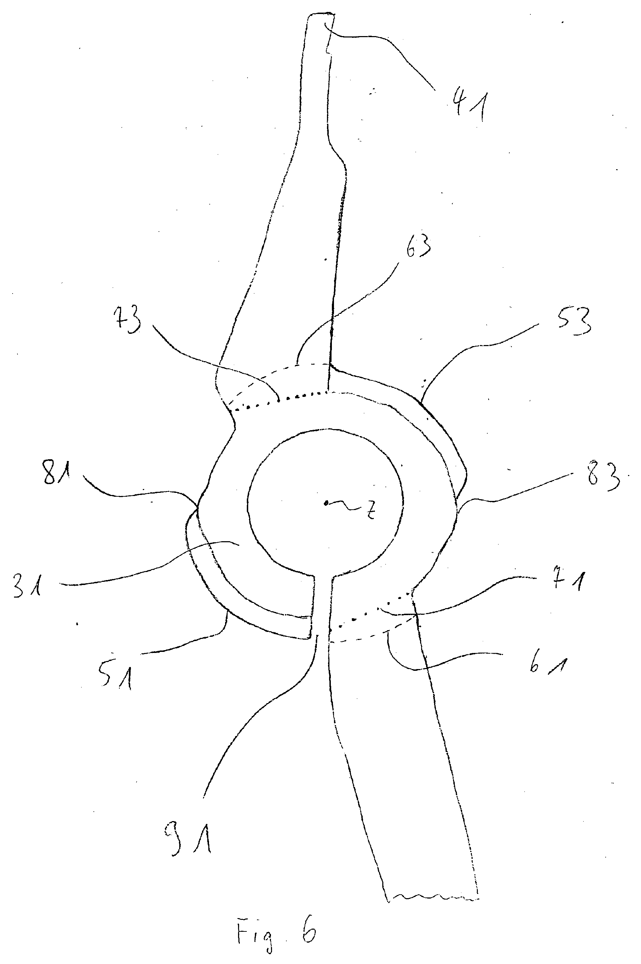

- Fig. 6: eine vergrößerte Darstellung des Drehgelenkelements.

Das Ausführungsbeispiel der chirurgischen Zange gemäß den Figuren umfasst einen Griff 2, ein Drehgelenk 3, ein Maul 4 und ein erstes und ein zweites Zangenteil 11, 12. Das erste Zangenteil 11 umfasst ein erstes Bedienelement 21, ein erstes Drehgelenkelement 31 und ein erstes Maulelement 41. Im gekoppelten Zustand, wenn das erste Zangenteil 11 mit einem zweiten Zangenteil 12 gekoppelt ist, kann über das erste Bedienelement 21 und ein zweites Bedienelement 22, das am zweiten Zangenteil 12 angeordnet ist, eine Kraft über das erste Drehgelenkelement 31 und ein zweites Drehgelenkelement 32 auf das erste Maulelement 41 und ein zweites Maulelement 42 übertragen werden. Das erste und das zweite Maulelement 41, 42 können entweder als Scherenklingen ausgestaltet sein oder als Zangenmäuler. Das erste Drehgelenkelement 31 weist eine erste Führungsschiene 51 sowie ein erstes Verdickungselement 81 auf. Das erste Verdickungselement 81 kann in einer Ausführungsform auf der Führungsschiene 51 angeordnet sein und in einer anderen Ausführungsform neben der Führungsschiene 51 angeordnet sein. Weiter umfasst das erste Drehgelenkelement eine erste Führungsausnehmung 61 sowie ein erstes Oberflächenelement 71. In einer Ausführungsform ist das erste Oberflächenelement 71 neben der ersten Führungsausnehmung 61 angeordnet und in einer anderen Ausführungsform in der Führungsausnehmung 61 angeordnet.

Das zweite Zangenteil 12 umfasst ein zweites Bedienelement 22, ein zweites Drehgelenkelement 32 und ein zweites Maulelement 42. Weiter weist es eine zweite Führungsschiene 52 auf sowie ein zweites Verdickungselement 82. Die Führungsausnehmungen 61, 62 bilden dabei einen Hinterschnitt aus, in dem die Führungsschienen 51, 52 im gekoppelten Zustand angeordnet sind, wobei sie eine Gleitführung für das Drehgelenk 3 ausbilden. Dabei ist die zweite Führungsschiene 52 in der ersten Führungsausnehmung 61 sowie die erste Führungsschiene 51 in der zweiten Führungsausnehmung 62 angeordnet. Die in den Führungsausnehmungen 61, 62 gelagerten Führungsschienen 51, 52 bewirken über den Hinterschnitt die Kopplung des ersten Zangenteils 11 mit dem zweiten Zangenteil 12. Weiter führen die Führungsschienen 51, 52 und die Führungsausnehmungen 61, 62 die beiden Zangenteile 11, 12 während einer Drehung um die zentrale Achse Z.

Das erste und das zweite Drehgelenkelement 31, 32 weisen weiter eine zentrale Achse Z auf, die im gekoppelten Zustand mit der Drehachse der Zangenteile 11, 12 zusammenfällt. Die Drehgelenkelemente 31, 32 sind als Ringfedern ausgebildet, wobei die Ringfedern auf der Seite der Drehgelenkelemente 31, 32, an der die Bedienelemente 21, 22 angeordnet sind, Schlitze 91, 92 aufweisen.

Die gekrümmten Flächen der Ringfeder sind in einer Ebene senkrecht zur Achse Z angeordnet. Dabei ist der erste Schlitz 91 des ersten Drehgelenkelements 31 am ersten Bedienelement 21 angeordnet und der zweite Schlitz 92 des zweiten Drehgelenkelements 32 am zweiten Bedienelement 22 angeordnet. Während der Kopplung zwischen dem ersten Drehgelenkelement 31 und dem zweiten Drehgelenkelement 32 sind beide Drehgelenkelemente 31, 32 immer noch gegeneinander drehbar um die Achse Z gelagert. Die Schlitze 91, 92 ermöglichen in der Endposition eine Federwirkung auf die Bedienelemente 21, 22. Dadurch wird eine erhöhte Krafteinwirkung auf die Bedienelemente 21, 22 abgefedert, so dass die beiden Maulelemente 41, 42 nur unwesentlich stärker zusammengedrückt werden.

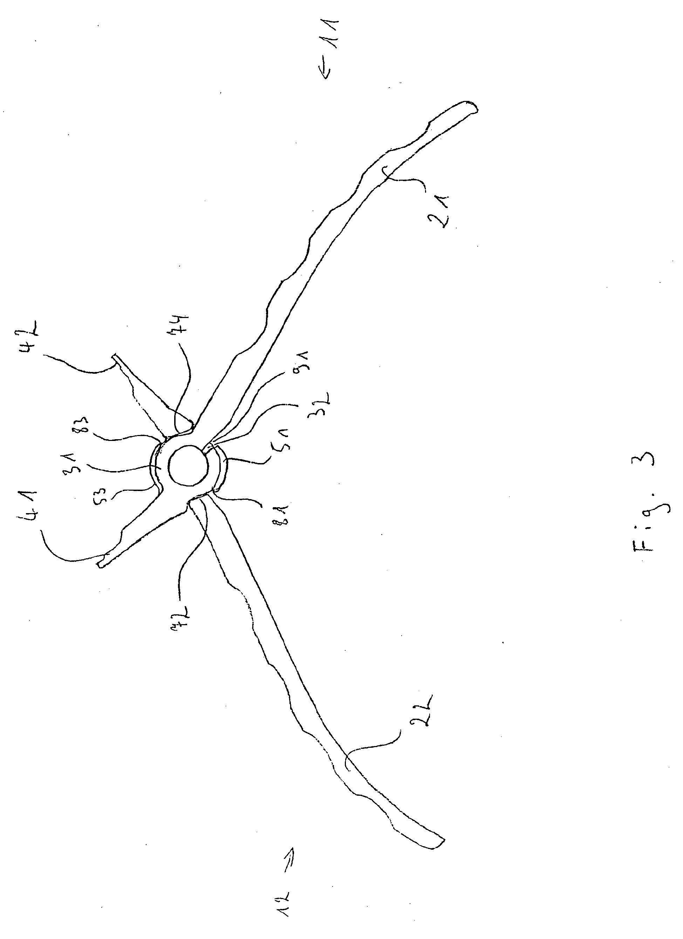

In Figur 3 wird die Öffnungsposition der beiden Zangenteile 11, 12 dargestellt. In dieser Öffnungsposition sind die beiden Zangenteile 11, 12 lösbar aneinander angeordnet. Die Führungsschienen 51, 52 sind nicht in den Führungsausnehmungen 61, 62 angeordnet. Dies ermöglicht die Lösung der beiden Zangenteile 11, 12. Weiter ist das erste Verdickungselement 81 nicht in Kontakt mit dem zweiten Oberflächenelement 72. Das zweite Verdickungselement 82 ist ebenfalls nicht in Kontakt mit dem ersten Oberflächenelement 71. Durch eine Drehung der beiden Zangenteile 11, 12 um die Achse Z werden die Führungsschienen 51, 52 in die Führungsausnehmungen 61, 62 eingeschoben. Dabei wird die erste Führungsschiene 51 des ersten Zangenteils 11 in die zweite Führungsausnehmung 62 des zweiten Zangenteils 12 eingeführt. Die zweite Führungsschiene 52 des zweiten Zangenteils 12 wird in die erste Führungsausnehmung 61 des ersten Zangenteils 11 eingeführt. Weiter kommt das erste Verdickungselement 81 des ersten Zangenteils 11 mit dem zweiten Oberflächenelement 72 des zweiten Zangenteils 12 in Kontakt. Das zweite Verdickungselement 82 des zweiten Zangenteils 12 kommt in Kontakt mit dem ersten Oberflächenelement 71 des ersten Zangenteils 11.

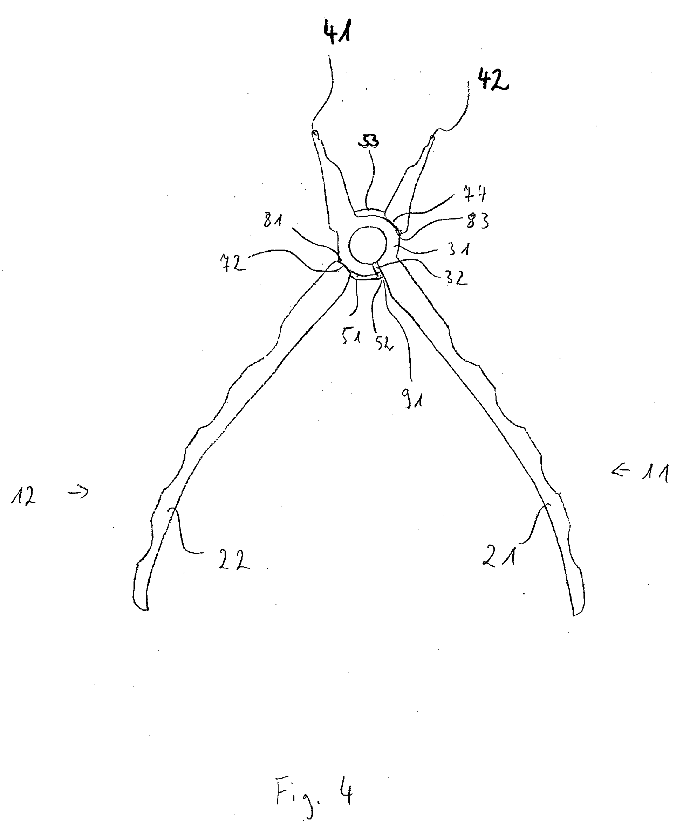

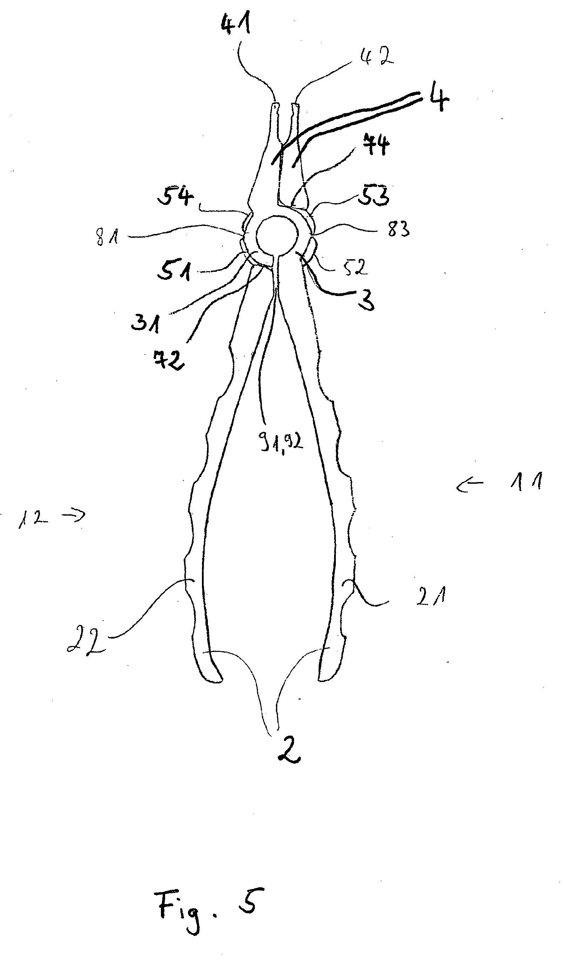

Dabei erreicht man die in Figur 4 gezeigte Sicherungsposition des Werkzeugs. In dieser Position wirkt das erste Verdickungselement 81 mit dem zweiten Oberflächenelement 72 und das zweite Verdickungselement 82 mit dem ersten Oberflächenelement 71 so zusammen, dass eine Verklemmung auftritt. Dadurch wird eine Drehung der beiden Zangenteile 11 und 12 gegeneinander um die Achse Z erschwert. Nur mit einem erhöhten Kraftaufwand ist es möglich, eine weitere Drehung zu erreichen. Ist die Drehung derart weit fortgeschritten, dass das erste Verdickungselement 81 nicht mehr in Kontakt mit dem zweiten Oberflächenelement 72 und das zweite Verdickungselement 82 mit dem ersten Oberflächenelement 71 steht, ist eine weitere Drehung ohne erhöhten Kraftaufwand bis zu der in Figur 5 gezeigten Endposition möglich. Aus der Endposition können die beiden Zangenteile 11 und 12 nur noch in Richtung der Sicherungsposition gegeneinander verdreht werden.

In einer vorteilhaften Ausführungsform umfasst das erste Zangenteil 11 ein erstes Drehgelenkelement 31 mit einem ersten Oberflächenelement 71, einer dritten Führungsschiene 53, einem dritten Verdickungselement 83, einem dritten Oberflächenelement 73 und einer dritten Führungsausnehmung 63. Dementsprechend umfasst in dieser Ausführungsform das zweite Zangenteil 12 ein Drehgelenkelement 32 mit einem zweiten Verdickungselement 82, einer vierten Führungsschiene 54, einem vierten Oberflächenelement 74 und einem vierten Verdickungselement 84 sowie einer vierten Führungsausnehmung 64. Die dritte Führungsausnehmung 63, die dritte Führungsschiene 53, das dritte Oberflächenelement 73 und das dritte Verdickungselement 83 sind derart angeordnet, dass sie aus einer Drehung der ersten Führungsausnehmung 61 bzw. der ersten Führungsschiene 51 bzw. dem ersten Oberflächenelement 71 bzw. des ersten Verdickungselements 81 hervorgehen. Die vierte Führungsausnehmung 64, das vierte Oberflächenelement 74 und die vierte Führungsschiene 54 sowie das vierte Verdickungselement 84 gehen aus einer Drehung um die Achse Z der zweiten Führungsausnehmung 62 bzw. dem zweiten Oberflächenelement 72 bzw. der zweiten Führungsschiene 52 bzw. des zweiten Verdickungselements 82 hervor. Die Führungsschienen 51, 52 und 53, 54 sind jeweils auf den Außenseiten der Ringfedern angeordnet. Die Führungsschienen 51, 52, 53, 54 sind dabei derart ausgestaltet, dass sie in Bezug zur Achse Z lediglich einen Winkel von höchstens 90° überstreichen.

In einer gekoppelten Position ist die dritte Führungsschiene 53 des ersten Drehgelenkelements 31 in der vierten Führungsausnehmung 64 angeordnet und die vierte Führungsschiene 54 des zweiten Drehgelenkelements 32 ist in der dritten Führungsausnehmung 63 des ersten Drehgelenkelements 31 angeordnet. In der Sicherungsposition wirkt das dritte Verdickungselement 83 des ersten Drehgelenkelements 31 mit dem vierten Oberflächenelement 74 zusammen. Das vierte Verdickungselement 84 des zweiten Drehgelenkelements 32 wirkt in dieser Position mit dem dritten Oberflächenelement 73 des ersten Drehgelenkelements 31 zusammen. Dadurch erhöht sich der Kraftaufwand, mit dem die beiden Zangenteile 11, 12 aus der Sicherungsposition verdreht werden können. Dies erhöht die Sicherheit der Handhabung des Werkzeugs erheblich.

In einer weiteren vorteilhaften Ausführungsform sind die beiden Zangenteile 11 und 12 identisch aufgebaut. Dadurch lassen sich die Herstellungskosten des Werkzeugs senken. Weiter hat dies den Vorteil, dass bei einem Verlust eines Zangenteils 11, 12 das jeweils verbleibende Zangenteil mit einem dritten Zangenteil der gleichen Bauart kombiniert werden kann. Das Gleiche gilt für eine Beschädigung eines Zangenteils 11, 12. Weiter können sie Ausnehmungen 101 für den Eingriff durch Finger aufweisen, durch die ein Abrutschen der Finger während der Bedienung verhindert wird.

Die Verdickungselemente 81, 82, 83, 84 können auf den Führungsschienen 51, 52, 53, 54 angeordnet sein sowie die Oberflächenelemente 71, 72, 73, 74 in den Führungsausnehmungen 61, 62, 63, 64 angeordnet sein. Ebenso können die Verdickungselemente 81, 82, 83, 84 in den Führungsausnehmungen 61, 62, 63, 64 angeordnet sein und die Oberflächenelemente 71, 72, 73, 74 auf den Führungsschienen 51, 52, 53, 54.

Das Material, aus dem das erste und das zweite Zangenteil 11, 12 bestehen, hat vorzugsweise eine nicht-korrodierende Eigenschaft und lässt nur eine sehr eingeschränkte Besiedlung mit Bakterien und Keimen zu. Derartige Materialien sind z. B. rostfreie Stähle, nicht-korrodierende Legierungen, oder auch spezielle Kunststoffe.

## Patentansprüche

1. Zange, die einen Griff (2), ein Drehgelenk (3) mit einer Drehachse und ein Maul (4) umfasst, wobei sie zwei lösbar gekoppelte Zangenteile (11, 12) umfasst, die jeweils ein zapfenloses Drehgelenkelement (31, 32) aufweisen, wobei das erste Drehgelenkelement (31) eine erste Führungsschiene (51) und eine erste Führungsausnehmung (61) aufweist, und das zweite Drehgelenkelement (32) eine zweite Führungsschiene (52) und zweite Führungsausnehmung (62) aufweist, wobei die in einen Hinterschnitt der Führungsausnehmungen (61, 62) an den Drehgelenkelementen (31, 32) greifenden Führungsschienen (51, 52) eine Gleitführung für das Drehgelenk (3) bilden,
**dadurch gekennzeichnet, dass**
das erste Drehgelenkelement (31) ein erstes Verdickungselement (81) aufweist und das zweite Drehgelenkelement (32) ein zweites Oberflächenelement (72) aufweist, wobei in einer Sicherungsposition das erste Verdickungselement (81) mit dem zweiten Oberflächenelement (72) durch eine Verklemmung zusammenwirkt.

2. Zange nach Anspruch 1,
**dadurch gekennzeichnet, dass**
die Zangenteile (11, 12) identisch ausgeführt sind.

3. Zange nach den Ansprüchen 1 und 2,
**dadurch gekennzeichnet, dass**
das erste Drehgelenkelement (31) ein erstes Oberflächenelement (71), eine dritte Führungsschiene (53), eine dritte Führungsausnehmung (63), ein drittes Oberflächenelement (73) und ein drittes Verdickungselement (83) aufweist und das zweite Drehgelenkelement (32) ein zweites Verdickungselement (82), eine vierte Führungsschiene (54), eine vierte Führungsausnehmung (64), ein viertes Oberflächenelement (74) und ein viertes Verdickungselement (84) aufweist.

4. Zange nach Anspruch 3,
**dadurch gekennzeichnet, dass**
in der Sicherungsposition das zweite Verdickungselement (82) mit dem ersten Oberflächenelement (71) und/oder das dritte Verdickungselement (83) mit dem vierten Oberflächenelement (74) und/oder das vierte Verdickungselement (84) mit dem dritten Oberflächenelement (73) durch eine Verklemmung zusammenwirkt.

5. Zange nach den Ansprüchen 1 bis 4,
**dadurch gekennzeichnet, dass**
die Bedienelemente (21, 22) Ausnehmungen (101) zum Eingriff durch Finger aufweisen.

6. Zange nach den Ansprüchen 1 bis 5,
**dadurch gekennzeichnet, dass**
das erste Drehgelenkelement (31) als geschlitzte Ringfeder ausgestaltet ist, wobei die Ringfeder einen ersten Schlitz (91) aufweist, der am Griff (2) angeordnet ist.

7. Zange nach den Ansprüchen 1 bis 6,
**dadurch gekennzeichnet, dass**
das zweite Drehgelenkelement (32) als geschlitzte Ringfeder ausgestaltet ist, wobei die Ringfeder einen zweiten Schlitz (92) aufweist, der am Griff (2) angeordnet ist.

8. Zange nach den Ansprüchen 1 bis 7,
**dadurch gekennzeichnet, dass**
die Führungsschienen (51, 52, 53, 54) in Bezug auf die Drehachse des Drehgelenks (3) jeweils höchstens einen Winkel von 90° überstreichen.

9. Zange nach den Ansprüchen 1 bis 8,
**dadurch gekennzeichnet, dass**
sie aus einem Material besteht, das korrosionsfrei ist und eine Besiedlung mit Bakterien erschwert, wobei das Material ein Metall, eine Legierung oder ein Kunststoff sein kann.

## Claims

1. Forceps comprising a handle (2), a pivot joint (3) with an axis of rotation, and a jaw (4), wherein the forceps comprise two detachably coupled forceps parts (11, 12) which each have a pinless pivot joint element (31, 32), wherein the first pivot joint element (31) has a first guide rail (51) and a first guide recess (61), and the second pivot joint element (32) has a second guide rail (52) and a second guide recess (62), wherein the guide rails (51, 52) engaging in an undercut of the guide recesses (61, 62) on the pivot joint elements (31, 32) form a slide guide for the pivot joint (3),
**characterized in that**
the first pivot joint element (31) has a first thickening element (81), and the second pivot joint element (32) has a second surface element (72), wherein the first thickening element (81) interacts with the second surface element (72) by clamping in a safety position.

2. Forceps according to Claim 1,
**characterized in that**
the forceps parts (11, 12) are designed identically.

3. Forceps according to Claims 1 and 2,
**characterized in that**
the first pivot joint element (31) has a first surface element (71), a third guide rail (53), a third guide recess (63), a third surface element (73) and a third thickening element (83), and the second pivot joint element (32) has a second thickening element (82), a fourth guide rail (54), a fourth guide recess (64), a fourth surface element (74) and a fourth thickening element (84).

4. Forceps according to Claim 3,
**characterized in that**
in the safety position the second thickening element (82) interacts with the first surface element (71) and/or the third thickening element (83) interacts with the fourth surface element (74) and/or the fourth thickening element (84) interacts with the third surface element (73) by clamping.

5. Forceps according to Claims 1 to 4,
**characterized in that**
the control elements (21, 22) have recesses (101) for engagement by fingers.

6. Forceps according to Claims 1 to 5,
**characterized in that**
the first pivot joint element (31) is designed as a slit annular spring, wherein the annular spring has a first slit (91) which is arranged on the handle (2).

7. Forceps according to Claims 1 to 6,
**characterized in that**
the second pivot joint element (32) is designed as a slit annular spring, wherein the annular spring has a second slit (92) which is arranged on the handle (2).

8. Forceps according to Claims 1 to 7,
**characterized in that**
the guide rails (51, 52, 53, 54) each cover at most an angle of 90° with respect to the axis of rotation of the pivot joint (3).

9. Forceps according to Claims 1 to 8,
**characterized in that**
they are made of a material that is corrosion-free and makes colonization of bacteria difficult, wherein the material can be a metal, an alloy or a plastic.

## Revendications

1. Pince comprenant une poignée (2), une articulation pivotante (3) munie d'un axe de rotation et une mâchoire (4), la pince comprenant deux parties de pince (11, 12) accouplées de manière amovible, lesquelles présentent chacune un élément d'articulation pivotante sans tourillon (31, 32), le premier élément d'articulation pivotante (31) présentant un premier rail de guidage (51) et un premier évidement de guidage (61), et le deuxième élément d'articulation pivotante (32) présentant un deuxième rail de guidage (52) et un deuxième évidement de guidage (62), les rails de guidage (51, 52) s'engageant dans une contre-dépouille des évidements de guidage (61, 62) au niveau des éléments d'articulation pivotante (31, 32) formant un guide coulissant pour l'articulation pivotante (3),
**caractérisée en ce que**
le premier élément d'articulation pivotante (31) présente un premier élément d'épaississement (81) et le deuxième élément d'articulation pivotante (32) présente un deuxième élément de surface (72), le premier élément d'épaississement (81), dans une position de fixation, coopérant avec le deuxième élément de surface (72) par un système de serrage.

2. Pince selon la revendication 1,
**caractérisée en ce que**
les parties de pince (11, 12) sont réalisées de manière identique.

3. Pince selon les revendications 1 et 2,
**caractérisée en ce que**
le premier élément d'articulation pivotante (31) présente un premier élément de surface (71), un troisième rail de guidage (53), un troisième évidement de guidage (63), un troisième élément de surface (73) et un troisième élément d'épaississement (83) et le deuxième élément d'articulation pivotante (32) présente un deuxième élément d'épaississement (82), un quatrième rail de guidage (54), un quatrième évidement de guidage (64), un quatrième élément de surface (74) et un quatrième élément d'épaississement (84).

4. Pince selon la revendication 3,
**caractérisée en ce que**
dans la position de fixation, le deuxième élément d'épaississement (82) coopère avec le premier élément de surface (71) et/ou le troisième élément d'épaississement (83) coopère avec le quatrième élément de surface (74) et/ou le quatrième élément d'épaississement (84) coopère avec le troisième élément de surface (73) par un système de serrage.

5. Pince selon les revendications 1 à 4,
**caractérisée en ce que**
les éléments de commande (21, 22) présentent des évidements (101) pour l'engagement par des doigts.

6. Pince selon les revendications 1 à 5,
**caractérisée en ce que**
le premier élément d'articulation pivotante (31) est réalisé sous forme de ressort annulaire fendu, le ressort annulaire présentant une première fente (91) qui est disposée sur la poignée (2).

7. Pince selon les revendications 1 à 6,
**caractérisée en ce que**
le deuxième élément d'articulation pivotante (32) est réalisé sous forme de ressort annulaire fendu, le ressort annulaire présentant une deuxième fente (92) qui est disposée sur la poignée (2).

8. Pince selon les revendications 1 à 7,
**caractérisée en ce que**
les rails de guidage (51, 52, 53, 54) couvrent à chaque fois au maximum un angle de 90° par rapport à l'axe de rotation de l'articulation pivotante (3).

9. Pince selon les revendications 1 à 8,
**caractérisée en ce**
**qu'**elle se compose d'un matériau inoxydable qui rend plus difficile une colonisation par des bactéries, le matériau pouvant être un métal, un alliage ou un plastique.
